# EUROPEAN PATENT APPLICATION

(11) **EP 4 706 517 A1**
(43) Date of publication of application: **11.03.2026**
(21) Application number: 24819291.6
(22) Date of filing: 03.06.2024
(51) Int. Cl.: A61B 5/022, A61B 5/02

(54) **SPHYGMOMANOMETER, BLOOD PRESSURE MEASUREMENT METHOD, BLOOD PRESSURE MEASUREMENT PROGRAM, LEARNING MODEL CONSTRUCTION METHOD, AND LEARNING MODEL CONSTRUCTION PROGRAM**

(30) Priority: 08.06.2023 JP 2023094838
(71) Applicant: OMRON Corporation, Kyoto-shi, Kyoto 600-8530 (JP); Omron Healthcare Co., Ltd., Muko-shi, Kyoto 617-0002 (JP)
(72) Inventor: UCHIDA, Shihori, Kyoto-shi, Kyoto 600-8530 (JP)
(74) Representative: Isarpatent
(86) International application number: PCT/JP2024/020192
(87) International publication number: WO 2024/253061

(57) **Abstract**

The sphygmomanometer includes: a cuff wound around an arm of a measurement subject; a control unit configured to control a pressure applied to the arm by the cuff; a detection unit configured to detect a cuff pressure; a generation unit configured to generate a first waveform image that indicates the cuff pressure; a calculation model constructed by machine learning in which a second waveform image that includes a second image indicating a change in pressure brought by pulsation of a blood flow flowing through an artery of a subject, and that indicates the cuff pressure with features of the second image is used as an explanatory variable, and a blood pressure of the subject is used as a response variable; and an acquisition unit configured to acquire a blood pressure of the measurement subject from the calculation model by inputting the first waveform image to the calculation model.

## Description

### TECHNICAL FIELD

The present invention relates to a sphygmomanometer, a blood pressure measurement method, a blood pressure measurement program, a learning model construction method, and a learning model construction program.

### BACKGROUND OF INVENTION

A health condition is grasped by blood pressure measurement. PTL 1 describes a technique in which a correlation between a blood pressure of a measurement subject measured using a cuff and a blood pressure of the measurement subject actually measured by an auscultation method is stored, and the blood pressure of the measurement subject is corrected using the stored correlation. PTL 2 describes a technique for measuring blood pressure using photoplethysmography. PTL 3 proposes a technique for measuring blood pressure using an image of features generated by performing wavelet transform or the like on a cuff pressure pulse wave.

### CITATION LIST

### PATENT LITERATURE

PTL 1: JP 5-309073 A
PTL 2: JP 2022-516820 T
PTL 3: US 2020/146568 A

### SUMMARY

### TECHNICAL PROBLEM

Pulse waves, photoplethysmographic waves, and the like obtained from cuff pressure include noise in some cases depending on the body motion of a measurement subject, the way of winding the cuff, the position where the cuff is wound, and the like. In the blood pressure measurement by the oscillometric method, blood pressure is measured based on the waveform of a pulse wave; due to this, in a case where the above-mentioned noise is included in the pulse wave, the accuracy of the blood pressure measurement may be degraded.

An object of one aspect of the disclosed technique is to provide a sphygmomanometer, a blood pressure measurement method, a blood pressure measurement program, a learning model construction method, and a learning model construction program capable of acquiring blood pressure with high accuracy even when noise is included in the pulse wave.

### SOLUTION TO PROBLEM

One aspect of the disclosed technique is exemplified by a sphygmomanometer given below. The sphygmomanometer includes: a cuff configured to be wound around an arm of a measurement subject; a control unit configured to control a pressure applied to the arm by the cuff; a detection unit configured to detect a cuff pressure received by the cuff from the arm; a generation unit configured to generate a first waveform image that includes a first image of a first pulse waveform acquired from a time-series change in the cuff pressure and indicating a change in pressure brought by pulsation of a blood flow flowing through an artery of the measurement subject, and that indicates the cuff pressure with features of the first image; a calculation model constructed by machine learning in which a second waveform image that includes a second image of a second pulse waveform acquired from a time-series change in the cuff pressure regarding a subject and indicating a change in pressure brought by pulsation of a blood flow flowing through an artery of the subject, and that indicates the cuff pressure with features of the second image is used as an explanatory variable, and a blood pressure of the subject is used as a response variable; and an acquisition unit configured to acquire a blood pressure of the measurement subject from the calculation model by inputting the first waveform image to the calculation model.

The time-series change in the cuff pressure includes a time-series change in pressure brought by pulsation of a blood flow flowing through an artery of the arm being superimposed as minute vibrations on the time-series change in the pressure with which the cuff presses the arm. According to the sphygmomanometer, the waveform image includes a waveform indicating a global change and a waveform indicating a local change of the pulse waveform in which the time-series change in pressure brought by pulsation of the blood flow flowing through the artery of the arm indicates minute vibrations, and also includes information of the cuff pressure. The calculation model constructed by the machine learning taking the above-discussed waveform image as a response variable can output the blood pressure of the measurement subject with high accuracy even when the waveform image includes noise. That is, according to the sphygmomanometer, even when the pulse wave obtained from the cuff pressure includes noise, highly accurate blood pressure measurement can be performed. The arm of the measurement subject includes the wrist of the measurement subject.

The sphygmomanometer may further include the following features. The generation unit generates the first waveform image in which the pulse waveform is drawn in a form of a line image and the cuff pressure is indicated with features of a line forming the line image. Such a waveform image can include both global features and local features of the pulse waveform due to the pulse waveform being drawn in the form of a line image, and can also include the information of the cuff pressure by the features of the line. In this case, the features of the line may include a color of the line, and the cuff pressure may be indicated by the color of the line. The features of the line may include a thickness of the line, and the cuff pressure may be indicated by the thicknesses of the line. By using such a waveform image, the sphygmomanometer can perform blood pressure measurement with higher accuracy.

The sphygmomanometer may further include the following features. The generation unit generates the first waveform image in which the pulse waveform is drawn in a form of a line image and the cuff pressure is indicated with features of the background of the line image. Such a waveform image can include both global features and local features of the pulse waveform due to the pulse waveform being drawn in the form of a line image, and can also include the information of the cuff pressure by the features of the background. In this case, the features of the background may include a color of the background, and the cuff pressure may be indicated by the color of the background. By using such a waveform image, the sphygmomanometer can perform blood pressure measurement with higher accuracy.

The sphygmomanometer may further include the following features. The calculation model is constructed by the machine learning in which the diastolic blood pressure and the systolic blood pressure of the subject are used as the response variables. The above-discussed calculation model makes it possible to learn the features effective for calculating the diastolic blood pressure and the systolic blood pressure.

The disclosed technique can also be understood from the aspects of a blood pressure measurement method, a blood pressure measurement program, a learning model construction method, and a learning model construction program.

### ADVANTAGEOUS EFFECTS OF INVENTION

According to the disclosed technique, it is possible to acquire blood pressure with high accuracy even when noise is included in a pulse wave.

### BRIEF DESCRIPTION OF THE DRAWINGS

FIG. 1 is a diagram illustrating an example of a configuration of a sphygmomanometer according to an embodiment.
FIG. 2 is a diagram schematically illustrating a state in which the sphygmomanometer is worn on a wrist of a measurement subject.
FIGS. 3A to 3D are diagrams exemplifying variations of waveform images.
FIG. 4 is a diagram illustrating a verification result of measurement accuracy of blood pressure when the blood pressure is measured using waveform images.
FIG. 5 is a diagram illustrating an example of a construction process of a blood pressure model according to the embodiment.
FIG. 6 is a diagram illustrating an example of a processing flow of the sphygmomanometer according to the embodiment.
FIG. 7 is a diagram illustrating an example of a configuration of a sphygmomanometer according to a comparative example.
FIG. 8 is a diagram exemplifying a comparison and verification result.

### DESCRIPTION OF EMBODIMENTS

### <Application Example>

An application example of the present invention will be described. A sphygmomanometer 100 according to the application example is a device configured to measure the blood pressure of a measurement subject by winding a cuff 120 around an upper arm or a wrist of the measurement subject. In the sphygmomanometer 100, an acquisition unit 114 inputs at least one of a time-series change in a cuff pressure and a time-series change in a pulse wave to a blood pressure model 116, and acquires the blood pressure of the measurement subject from the blood pressure model 116.

The acquisition unit 114 controls the pressure of the cuff 120 to acquire the time-series change in the cuff pressure and the time-series change in the pulse wave from a pressure sensor 113. The generation unit 115 generates a waveform image indicating the time-series change in the cuff pressure and the time-series change in the pulse wave acquired by the acquisition unit 114. The waveform image is obtained by, for example, adding the information indicating the time-series change in the cuff pressure to a line image indicating, with a line, the time-series change in the pulse wave by using features of the line image.

The acquisition unit 114 inputs the waveform image generated by the generation unit 115 to the blood pressure model 116 to acquire the blood pressure of the measurement subject from the blood pressure model 116. The acquisition unit 114 causes an output unit 117 to output the acquired blood pressure.

The blood pressure model 116 is a learning model that outputs, when the waveform image is input thereto, the blood pressure of the corresponding measurement subject. The blood pressure model 116 is constructed by machine learning in which the waveform image of the measurement subject is used as an explanatory variable and the blood pressure adopted as a true value is used as a response variable. The machine learning includes deep learning. The blood pressure adopted as a true value may be a blood pressure measured by an auscultation method.

According to the present application example, the waveform image includes a waveform indicating a global change and a waveform indicating a local change of the time-series change in the pulse wave. The blood pressure model 116 constructed by the machine learning taking the above-discussed waveform image as a response variable can output the blood pressure of the measurement subject with high accuracy even when the waveform image includes noise. That is, according to the present application example, even when noise is included in the waveform image generated by the generation unit 115, the blood pressure can be acquired with high accuracy.

### <Embodiments>

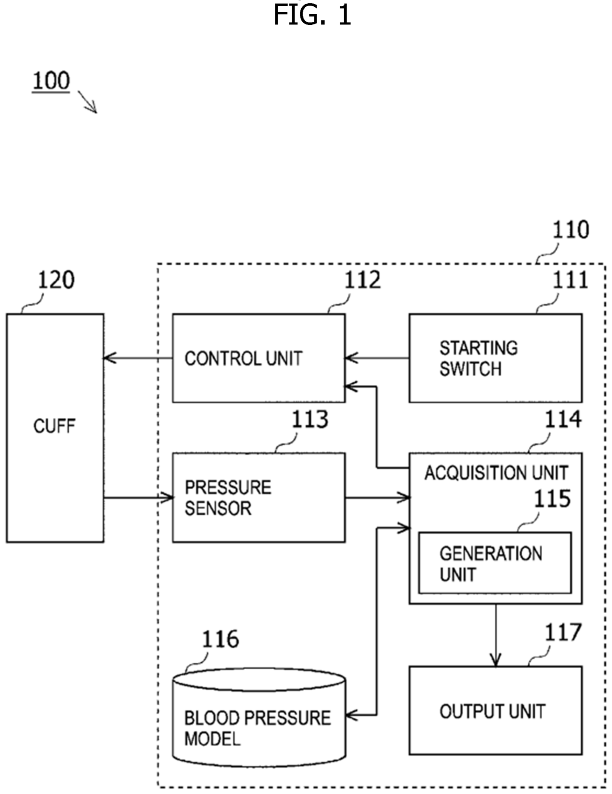

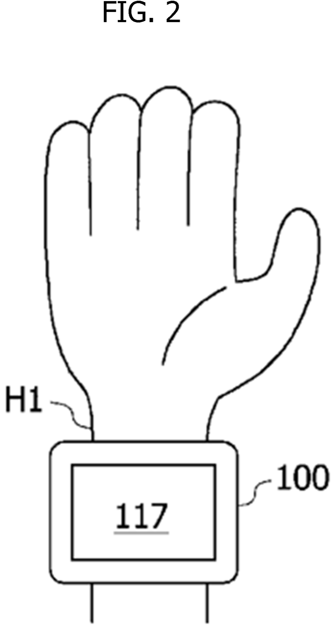

An embodiment will be described below with reference to the drawings. FIG. 1 is a diagram illustrating an example of a configuration of the sphygmomanometer 100 according to an embodiment. FIG. 2 is a diagram schematically illustrating a state in which the sphygmomanometer 100 is worn on a wrist H1 of a measurement subject. The sphygmomanometer 100 includes a main body 110 and the cuff 120. The sphygmomanometer 100 acquires the blood pressure of the measurement subject by, for example, winding the cuff 120 around the wrist H1 of the measurement subject. The position where the cuff 120 is wound is not limited to the wrist H1, and may be an upper arm of the measurement subject.

The cuff 120 is formed in a belt shape and includes a bag into which air is supplied. When air is supplied to the bag inside the cuff 120 in a state where the cuff 120 is wound around the wrist H1 of the measurement subject, the pressure of the cuff against the wrist H1 rises. When the air is sucked from the bag inside the cuff 120 in the state where the cuff 120 is wound around the wrist H1 of the measurement subject, the pressure of the cuff against the wrist H1 drops.

For example, a processor and a storage unit are disposed in the main body 110, and the processor executes a program stored in the storage unit, whereby respective processing units such as a starting switch 111, a control unit 112, the pressure sensor 113, the acquisition unit 114, the blood pressure model 116, and the output unit 117 are implemented. The acquisition unit 114 includes a generation unit 115. In other words, the sphygmomanometer 100 may be regarded as a computer including the cuff 120. Each of the processing units such as the starting switch 111, the control unit 112, the pressure sensor 113, the acquisition unit 114, the blood pressure model 116, and the output unit 117 may be a dedicated hardware circuit. As for the sphygmomanometer 100, when the starting switch 111 is turned on, the sphygmomanometer 100 starts to measure the blood pressure.

The control unit 112 controls the pressure applied to the wrist H1 of the measurement subject by the cuff 120, by supplying air to the cuff 120 and sucking air from the cuff 120. When the starting switch 111 is turned on, the control unit 112 starts controlling the pressure of the cuff 120.

The pressure sensor 113 is a sensor configured to detect the pressure (cuff pressure) by the cuff 120. The pressure sensor 113 outputs the detected cuff pressure to the acquisition unit 114. The time-series change in the cuff pressure includes a time-series change in pressure (pressure vibrations) brought by pulsation of a blood flow flowing through an artery of the wrist H1 being superimposed as minute vibrations on the time-series change in the pressure with which the cuff 120 presses the wrist H1.

The acquisition unit 114 acquires the blood pressure of the measurement subject by using the blood pressure model 116. The acquisition unit 114 acquires, from the pressure sensor 113, a time-series change in the cuff pressure of the cuff 120, which is increased to a pressure higher than the maximum blood pressure and then gradually decreased to a pressure lower than the minimum blood pressure. The acquisition unit 114 extracts a time-series change in pressure brought by the pulsation of the blood flow flowing through the artery from the time-series change in the cuff pressure acquired from the pressure sensor 113, and acquires a waveform indicating a time-series change in a pulse wave (hereinafter, also referred to as a pulse waveform).

The generation unit 115 generates one waveform image indicating the extracted time-series change in the cuff pressure and the pulse waveform. As described in the application example, the waveform image may be an image that draws a pulse waveform with a line and adds the information indicating the time-series change in the cuff pressure by using the features of the drawn line image. It is preferable for the waveform image to be generated in an uncompressed image file format. The image size of the waveform image preferably takes an image size that allows the pulse waveform to be visually recognized. The image size of the waveform image is, for example, 448 pixels in a longitudinal direction by 448 pixels in a lateral direction.

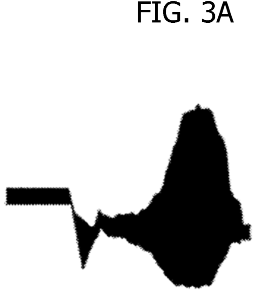

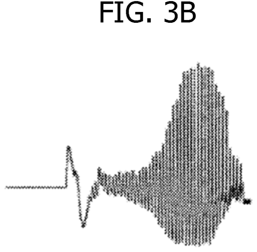

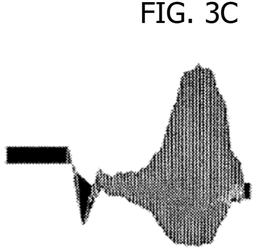

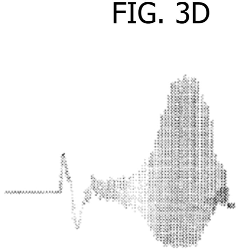

The waveform image will now be described. FIGS. 3A to 3D are diagrams exemplifying variations of waveform images. In each of FIGS. 3A, 3B, 3C, and 3D, the vertical axis exemplifies pressure (mmHg), while the horizontal axis exemplifies time (seconds). FIG. 3A exemplifies a waveform image generated by painting out a pulse waveform. FIG. 3B exemplifies a waveform image generated by drawing a pulse waveform with a line. FIG. 3C exemplifies a waveform image in which a waveform of a time-series change in a cuff pressure (hereinafter, also referred to as a cuff pressure waveform) is superimposed with a line on an image generated by painting out a pulse waveform. FIG. 3D exemplifies a waveform image in which a pulse waveform is drawn with a line and a time-series change in a cuff pressures is added by changing the color of the line in accordance with the cuff pressure.

The waveform image of FIG. 3A includes a variation in a global waveform shape such as an envelope shape of the pulse waveform. However, since the waveform image of FIG. 3A is painted out, information of a local variation in the pulse waveform is not included. The waveform image of FIG. 3B includes both a global waveform shape and a local waveform shape of the pulse waveform because the pulse waveform is drawn with a line. However, none of the waveform images of FIGS. 3A and 3B include information of a time-series change in the cuff pressure. The waveform image of FIG. 3C includes information of the time-series change in the cuff pressure, but does not include information of a local variation in the pulse waveform as in FIG. 3A. Because of this, in order to perform highly accurate blood pressure measurement, it is considered to be not preferable to use the waveform images exemplified in FIGS. 3A, 3B, and 3C for the blood pressure measurement.

The waveform image of FIG. 3D includes both a global waveform shape and a local waveform shape of the pulse waveform because the pulse waveform is drawn with the line. Further, in the waveform image of FIG. 3D, information indicating the time-series change in the cuff pressure is indicated by the color of the line forming the pulse waveform of the pulse wave. That is, FIG. 3D includes both the information indicating the time-series change in the pulse waveform and the information indicating the time-series change in the cuff pressure.

Hereinafter, the accuracy of the blood pressure measurement is verified when the measurement is performed using the waveform images respectively exemplified in FIGS. 3A, 3B, 3C, and 3D. In the verification, systolic blood pressures (SBP) were measured using the waveform images respectively exemplified in FIGS. 3A, 3B, 3C, and 3D. In the verification, the measurement of the blood pressure was performed a plurality of times for each of a plurality of the subjects, thereby acquiring the measurement results. In this verification, the acquired measurement results are evaluated by the Standard Deviation of Error (SDE).

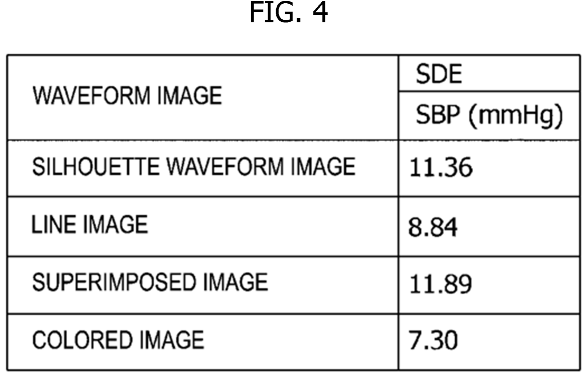

FIG. 4 is a diagram illustrating a verification result of the measurement accuracy of blood pressure when the blood pressure is measured using the waveform images. In FIG. 4, the waveform image exemplified in FIG. 3A is described as "silhouette image", the waveform image exemplified in FIG. 3B is described as "line image", the waveform image exemplified in FIG. 3C is described as "superimposed image", and the waveform image exemplified in FIG. 3C is described as "colored image". Referring to FIG. 4, the highest accuracy was exhibited when the blood pressure was measured using the "colored image". The reason for this can be considered as follows: since the "colored image" includes the information indicating the global waveform shape, the local waveform shape, and the time-series change in the cuff pressure of the pulse wave, the "colored image" has a larger amount of information than the other waveform images. In addition, it can also be considered that, due to the cuff pressure being indicated by the color of the line, the regularity of the relationship between the color information (RGB information) and the cuff pressure had a favorable influence on the accuracy of the blood pressure measurement.

As exemplified in FIG. 3D, the generation unit 115 generates a waveform image so that the information indicating the global waveform shape, the local waveform shape, and the time-series change in the cuff pressures of the pulse waveform is included in one waveform image. The generation unit 115 generates the waveform image that indicates the pulse waveform with a line image and indicates the information regarding the cuff pressure as features of the corresponding line, for example. Examples of features of the line indicating the information regarding the cuff pressure include the thickness of the line, the color of the line, the type of the line (a single line, a one-dot chain line, a two-dot chain line, a double line, or the like), and the brightness of the line. The generation unit 115 may generate the waveform image that indicates the pulse waveform in the form of a line image and indicates the information regarding the cuff pressure as features of the background of the pulse waveform, for example. Examples of the features of the background indicating the information regarding the cuff pressure include the color of the background and the brightness of the background.

The acquisition unit 114 inputs the waveform image generated by the generation unit 115 to the blood pressure model 116. The acquisition unit 114 acquires the diastolic blood pressure and the systolic blood pressure of the measurement subject as output from the blood pressure model 116 input with the waveform image.

The blood pressure model 116 is a learning model that outputs, when a waveform image is input thereto, the diastolic blood pressure and the systolic blood pressure. The blood pressure model 116 is stored in, for example, the storage unit housed in the main body 110. The blood pressure model 116 includes, for example, a deformable conv model.

The output unit 117 outputs the diastolic blood pressure and the systolic blood pressure acquired by the acquisition unit 114. The output unit 117 is, for example, a liquid crystal display (LCD), a plasma display panel (PDP), an inorganic electroluminescence (EL) panel, or an organic EL panel. The output unit 117 may be a printer or a speaker.

### <Construction of Blood Pressure Model 116>

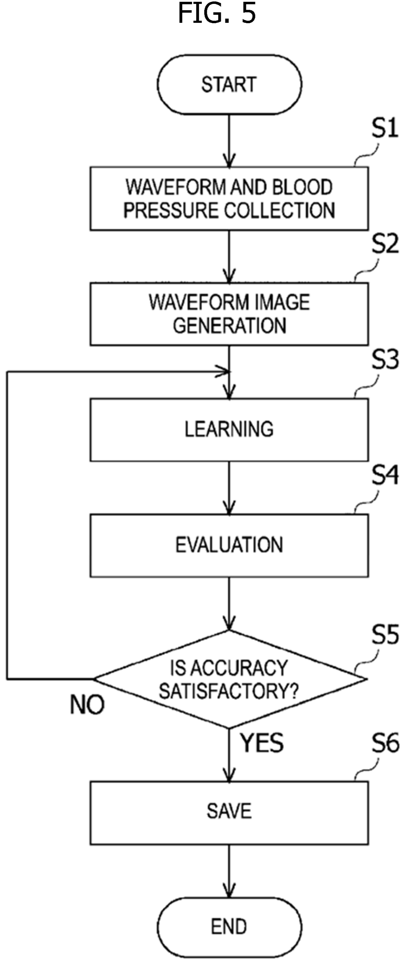

FIG. 5 is a diagram illustrating an example of a construction process of the blood pressure model 116 according to the embodiment. With reference to FIG. 5, an example of the construction process of the blood pressure model 116 will be described.

In step S1, the plurality of subjects are prepared. The plurality of subjects are prepared in the present embodiment, but the number of subjects to be prepared may be one. The cuff 120 is attached to each of the plurality of prepared subjects, and cuff pressure waveforms and pulse waveforms are collected. In addition, the diastolic blood pressure and the systolic blood pressure of the plurality of prepared subjects measured by the auscultation method are collected.

In step S2, the generation unit 115 generates a waveform image based on each of the cuff pressure waveforms and each of the pulse waveforms collected in step S1. In step S3, learning for constructing the blood pressure model 116 is executed by machine learning in which the waveform image generated in step S2 is used as an explanatory variable and the diastolic blood pressures and systolic blood pressures measured in step S1 are used as response variables.

In step S4, the blood pressure model 116 having been subjected to learning in step S3 is evaluated. In the evaluation, for example, a waveform image is generated for a certain subject, and the diastolic blood pressure and the systolic blood pressure are measured by the auscultation method. The generated waveform image is input to the blood pressure model 116, and the diastolic blood pressure and the systolic blood pressure are output from the blood pressure model 116. The diastolic blood pressure and the systolic blood pressure output by the blood pressure model 116 are compared with the diastolic blood pressure and the systolic blood pressure measured by the auscultation method to evaluate the blood pressure model 116.

As a result of the evaluation in step S4, when the blood pressure model 116 satisfies a desired accuracy (YES in step S5), the process proceeds to step S6. When the blood pressure model 116 does not satisfy the desired accuracy (NO in step S5), the process proceeds to step S3. In step S6, the generated blood pressure model 116 is stored in the storage unit of the sphygmomanometer 100.

### <Processing Flow>

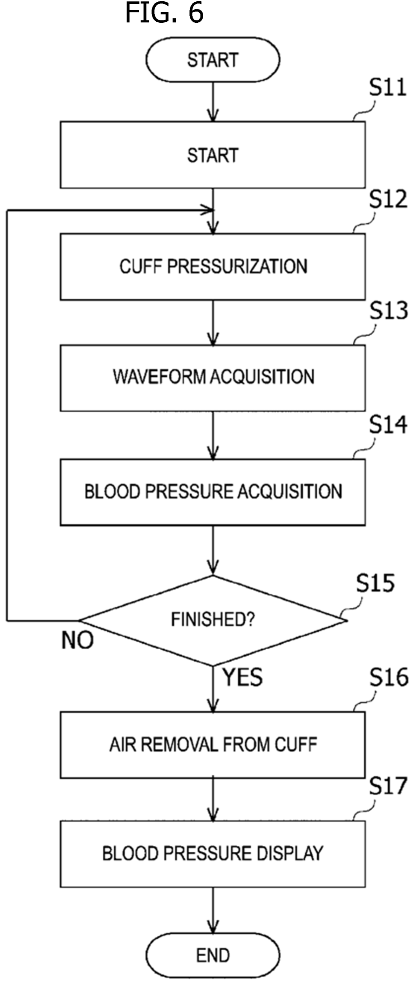

FIG. 6 is a diagram illustrating an example of a processing flow of the sphygmomanometer 100 according to the embodiment. Hereinafter, an example of the processing flow of the sphygmomanometer 100 will be described with reference to FIG. 6.

In step S11, the sphygmomanometer 100 is started when the starting switch 111 is turned on. In step S12, the control unit 112 supplies air to the cuff 120 to pressurize the wrist H1 of the measurement subject. The control unit 112 raises the pressure to a level higher than the maximum blood pressure by the cuff 120, and then gradually lowers the pressure of the cuff 120.

In step S13, the acquisition unit 114 acquires a cuff pressure waveform and a pulse waveform based on a cuff pressure detected by the pressure sensor 113. Further, the generation unit 115 generates a waveform image based on the waveform acquired in step S13. In step S14, the acquisition unit 114 inputs the waveform image generated in step S13 to the blood pressure model 116 to acquire the diastolic blood pressure and the systolic blood pressure of the measurement subject.

In step S15, the acquisition unit 114 determines whether the blood pressure measurement of the measurement subject is finished. The acquisition unit 114 may determine that the blood pressure acquisition is finished, for example, when both the diastolic blood pressure and the systolic blood pressure of the measurement subject have been acquired from the blood pressure model 116. When finished (YES in step S15), the process proceeds to step S16. When not finished (NO in step S15), the process proceeds to step S12.

In step S16, the control unit 112 removes the air from the cuff 120. In step S17, the output unit 117 outputs the blood pressures acquired in step S14.

### <Comparative Example>

In the embodiment, the blood pressure model 116 was constructed to output diastolic and systolic blood pressures. In a comparative example, a configuration in which the blood pressure model is divided into a blood pressure model that outputs a diastolic blood pressure and a blood pressure model that outputs a systolic blood pressure will be described.

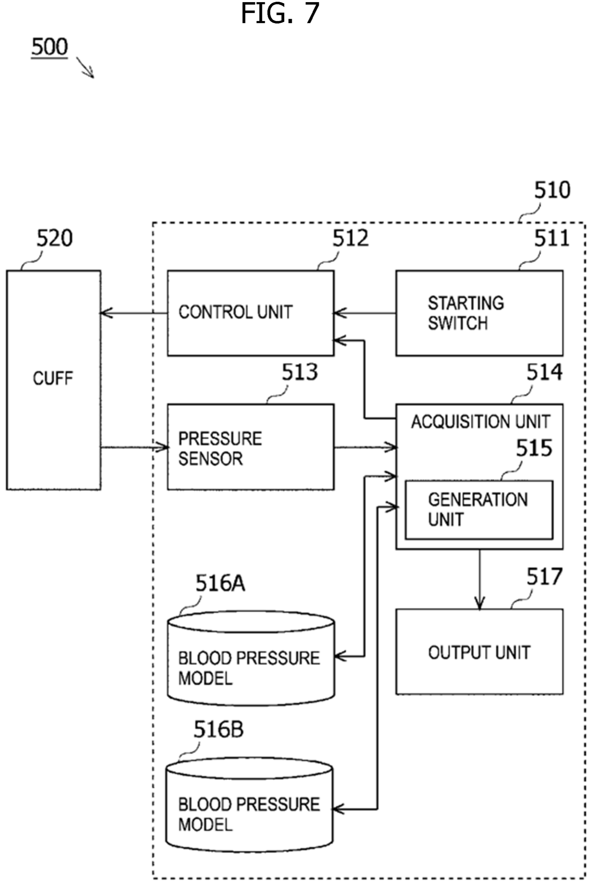

FIG. 7 is a diagram illustrating an example of a configuration of a sphygmomanometer 500 according to the comparative example. The sphygmomanometer 500 includes a main body 510, a starting switch 511, a control unit 512, a pressure sensor 513, an acquisition unit 514, a generation unit 515, a blood pressure model 516A, a blood pressure model 516B, an output unit 517, and a cuff 520. The main body 510, the starting switch 511, the control unit 512, the pressure sensor 513, the acquisition unit 514, the generation unit 515, and the cuff 520 are similar to the main body 110, the starting switch 111, the control unit 112, the pressure sensor 113, the acquisition unit 114, the generation unit 115, and the cuff 120 of the sphygmomanometer 100 according to the embodiment, and therefore the description thereof will be omitted.

The blood pressure model 516A is a learning model that outputs, when a waveform image is input thereto, the systolic blood pressure. The blood pressure model 516A is constructed by machine learning in which the waveform image is used as an explanatory variable and the systolic blood pressure adopted as a true value is used as a response variable. The blood pressure model 516B is a learning model that outputs, when a waveform image is input thereto, the diastolic blood pressure. The blood pressure model 516B is constructed by machine learning in which the waveform image is used as an explanatory variable and the diastolic blood pressure adopted as a true value is used as a response variable.

### <Comparison and Verification>

The blood pressure measurement accuracy of the sphygmomanometer 100 according to the embodiment and the blood pressure measurement accuracy of the sphygmomanometer 500 according to the comparative example were compared and verified, and the result thereof will be described below. In the comparison and verification, the systolic blood pressure (SBP) was measured with each of the sphygmomanometer 100 and the sphygmomanometer 500. In the comparison and verification, the measurement of the blood pressure was performed a plurality of times for each of the plurality of subjects, thereby acquiring the measurement results. In the comparison and verification, the acquired measurement results are evaluated by the SDE.

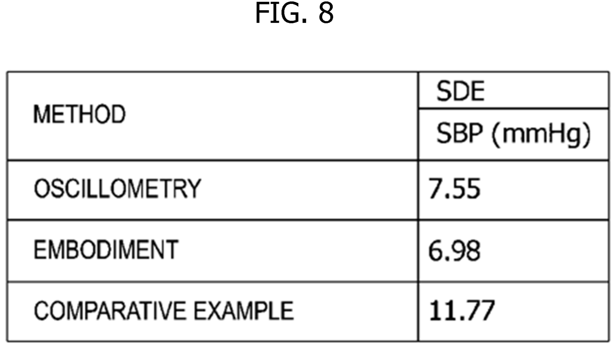

FIG. 8 is a diagram exemplifying the comparison and verification result. Referring to FIG. 8, the highest accuracy was exhibited when the blood pressure was measured using the "embodiment". From this, it is conceivable that higher measurement accuracy is obtained by simultaneously learning the systolic blood pressure and the diastolic blood pressure when constructing the blood pressure model.

### <Advantageous Effects of Embodiments>

According to the present embodiment, the waveform image generated by the generation unit 115 includes a waveform indicating a global change and a waveform indicating a local change of the time-series change in the pulse wave. The blood pressure model 116 constructed by the machine learning taking the above-discussed waveform image as a response variable can output the blood pressure of the measurement subject with high accuracy even when the waveform image includes noise. That is, according to the present application example, even when noise is included in the waveform image generated by the generation unit 115, the blood pressure can be measured with high accuracy.

In the present embodiment, a waveform image is generated in which a pulse waveform is drawn in a form of a line image and a cuff pressure is indicated by features of the line forming the line image. Such a waveform image can include both global features and local features of the pulse waveform due to the pulse waveform being drawn in the form of a line image, and can also include the information of the cuff pressure by the features of the line. By using such a waveform image, the sphygmomanometer 100 can perform blood pressure measurement with higher accuracy.

In the present embodiment, a waveform image may be generated in which a pulse waveform is drawn in a form of a line image and a cuff pressure is indicated by features of the background of the line image. Such a waveform image can include both global features and local features of the pulse waveform due to the pulse waveform being drawn in the form of a line image, and can also include the information of the cuff pressure by the features of the background. By using such a waveform image, the sphygmomanometer 100 can perform blood pressure measurement with higher accuracy.

In the present embodiment, the blood pressure model 116 learns the systolic blood pressure and the diastolic blood pressure at the same time. Therefore, the blood pressure model 116 can learn effective features in predicting both the systolic blood pressure and the diastolic blood pressure. Consequently, according to the present embodiment, the measurement of the systolic blood pressure and the diastolic blood pressure using the blood pressure model 116 can be made more accurate.

### <Modified Example>

In the waveform image of the embodiment described above, the information indicating a time-series change in the cuff pressure is added to a line image indicating the pulse waveform with a line, by using features of the line image. However, the waveform image is not limited to the above-described image. The waveform image may be an image in which, for example, information indicating a time-series change in the pulse wave is added to a line image indicating the cuff pressure waveform with a line.

The embodiments and modified examples disclosed above can be combined.

### <Computer-Readable Recording Medium>

An information processing program for causing a computer or any other machine or device (hereinafter referred to as a computer or the like) to implement any of the functions described above may be recorded in a recording medium readable by the computer or the like. The functions can be provided by causing the computer or the like to read and execute the program from the recording medium.

Here, the term "computer-readable recording medium" refers to a recording medium that stores information such as data or programs by electrical, magnetic, optical, mechanical, or chemical means, and that can be read by a computer or the like. Examples of such a recording medium that is removable from the computer or the like include a flexible disk, a magnetooptical disk, a compact disc read only memory (CD-ROM), a compact disc recordable (CD-R), a compact disc rewritable (CD-RW), a digital versatile disc (DVD), a Blu-ray disc (BD), a digital audio tape (DAT), an 8-mm tape, a flash memory, an external hard disk drive, and a solid state drive (SSD). Examples of a recording medium fixed to the computer or the like include a built-in type hard disk drive, an SSD, and a ROM.

### <Supplementary Note 1>

A sphygmomanometer (100) includes:
a cuff (120) configured to be wound around an arm of a measurement subject;
a control unit (112) configured to control a pressure applied to the arm (H1) by the cuff (120);
a detection unit (113) configured to detect a cuff pressure received by the cuff (120) from the arm (H1);
a generation unit (115) configured to generate a first waveform image that includes a first image of a first pulse waveform acquired from a time-series change in the cuff pressure and indicating a change in pressure brought by pulsation of a blood flow flowing through an artery of the measurement subject, and that indicates the cuff pressure with features of the first image;
a calculation model (116) constructed by machine learning in which a second waveform image that includes a second image of a second pulse waveform acquired from a time-series change in the cuff pressure regarding a subject and indicating a change in pressure brought by pulsation of a blood flow flowing through an artery of the subject, and that indicates the cuff pressure with features of the second image is used as an explanatory variable, and a blood pressure of the subject is used as a response variable; and
an acquisition unit (114) configured to acquire a blood pressure of the measurement subject from the calculation model (116) by inputting the first waveform image to the calculation model (116).

### <Supplementary Note 2>

The sphygmomanometer (100) according to supplementary note 1, in which
the generation unit (115) generates the first waveform image in which the pulse waveform is drawn in a form of a line image and the cuff pressure is indicated with features of a line forming the line image.

### <Supplementary Note 3>

The sphygmomanometer (100) according to supplementary note 2, in which
the features of the line include a color of the line, and
the cuff pressure is indicated by the color of the line.

### <Supplementary Note 4>

The sphygmomanometer (100) according to supplementary note 2 or 3, in which
the features of the line include a thickness of the line, and
the cuff pressure is indicated by the thickness of the line.

### <Supplementary Note 5>

The sphygmomanometer (100) according to supplementary note 1, in which
the generation unit (115) generates the first waveform image in which the pulse waveform is drawn in a form of a line image and the cuff pressure is indicated with features of a background of the line image.

### <Supplementary Note 6>

The sphygmomanometer (100) according to supplementary note 5, in which
the features of the background include a color of the background, and
the cuff pressure is indicated by the color of the background.

### <Supplementary Note 7>

The sphygmomanometer (100) according to any one of supplementary notes 1 to 6, in which
the calculation model (116) is constructed by the machine learning in which a diastolic blood pressure and a systolic blood pressure of the subject are each used as the response variable.

### <Supplementary Note 8>

A blood pressure measurement method includes:
by a computer (100),
a step of detecting a cuff pressure received by a cuff (120) configured to be wound around an arm (H1) of a measurement subject from the arm (H1 );
a step of generating a first waveform image that includes a first image indicating a pulse waveform acquired from a time-series change in the cuff pressure, and that indicates the cuff pressure with features of the first image; and
a step of acquiring a blood pressure of the measurement subject from a calculation model (116) by inputting the first waveform image to the calculation model (116), the calculation model (116) constructed by machine learning in which a second waveform image that includes a second image indicating a pulse waveform acquired from a time-series change in the cuff pressure regarding a subject, and that indicates the cuff pressure with features of the second image is used as an explanatory variable, and a blood pressure of the subject is used as a response variable.

### <Supplementary Note 9>

A blood pressure measurement program for causing a computer (100) to execute:
a step of detecting a cuff pressure received by a cuff (120) configured to be wound around an arm (H1) of a measurement subject from the arm (H1);
a step of generating a first waveform image that includes a first image indicating a pulse waveform acquired from a time-series change in the cuff pressure, and that indicates the cuff pressure with features of the first image; and
a step of acquiring a blood pressure of the measurement subject from a calculation model (116) by inputting the first waveform image to the calculation model (116), the calculation model (116) constructed by machine learning in which a second waveform image that includes a second image indicating a pulse waveform acquired from a time-series change in the cuff pressure regarding a subject, and that indicates the cuff pressure with features of the second image is used as an explanatory variable, and a blood pressure of the subject is used as a response variable.

### <Supplementary Note 10>

A learning model construction method includes:
by a computer (100),
a step of detecting a cuff pressure received by a cuff (120) configured to be wound around an arm of a subject from the arm;
a step of generating a waveform image that includes an image indicating a pulse waveform acquired from a time-series change in the cuff pressure, and that indicates the cuff pressure with features of the image; and
a step of constructing a learning model by machine learning in which the waveform image is used as an explanatory variable, and a blood pressure of the subject is used as a response variable.

### <Supplementary Note 11>

A learning model construction program for causing a computer (100) to execute:
a step of detecting a cuff pressure received by a cuff (120) configured to be wound around an arm of a subject from the arm;
a step of generating a waveform image that includes an image indicating a pulse waveform acquired from a waveform of a time-series change in the cuff pressure, and that indicates the cuff pressure with features of the image; and
a step of constructing a learning model by machine learning in which the waveform image is used as an explanatory variable, and a blood pressure of the subject is used as a response variable.

### REFERENCE SIGNS

100 Sphygmomanometer
110 Main body
111 Starting switch
112 Control unit
113 Pressure sensor
114 Acquisition unit
115 Generation unit
116 Blood pressure model
117 Output unit
120 Cuff
110 Main body
500 Sphygmomanometer
510 Main body
511 Starting switch
512 Control unit
513 Pressure sensor
514 Acquisition unit
515 Generation unit
516A Blood pressure model
516B Blood pressure model
517 Output unit
520 Cuff
H1 Wrist

## Claims

1. A sphygmomanometer comprising:
a cuff configured to be wound around an arm of a measurement subject;
a control unit configured to control a pressure applied to the arm by the cuff;
a detection unit configured to detect a cuff pressure received by the cuff from the arm;
a generation unit configured to generate a first waveform image that includes a first image of a first pulse waveform acquired from a time-series change in the cuff pressure and indicating a change in pressure brought by pulsation of a blood flow flowing through an artery of the measurement subject, and that indicates the cuff pressure with features of the first image;
a calculation model constructed by machine learning in which a second waveform image that includes a second image of a second pulse waveform acquired from a time-series change in the cuff pressure regarding a subject and indicating a change in pressure brought by pulsation of a blood flow flowing through an artery of the subject, and that indicates the cuff pressure with features of the second image is used as an explanatory variable, and a blood pressure of the subject is used as a response variable; and
an acquisition unit configured to acquire a blood pressure of the measurement subject from the calculation model by inputting the first waveform image to the calculation model.

2. The sphygmomanometer according to claim 1, wherein
the generation unit generates the first waveform image in which the pulse waveform is drawn in a form of a line image and the cuff pressure is indicated with features of a line forming the line image.

3. The sphygmomanometer according to claim 2, wherein
the features of the line include a color of the line, and
the cuff pressure is indicated by the color of the line.

4. The sphygmomanometer according to claim 2, wherein
the features of the line include a thickness of the line, and
the cuff pressure is indicated by the thickness of the line.

5. The sphygmomanometer according to claim 1, wherein
the generation unit generates the first waveform image in which the pulse waveform is drawn in a form of a line image and the cuff pressure is indicated with features of a background of the line image.

6. The sphygmomanometer according to claim 5, wherein
the features of the background include a color of the background, and
the cuff pressure is indicated by the color of the background.

7. The sphygmomanometer according to any one of claims 1 to 6, wherein
the calculation model is constructed by the machine learning in which a diastolic blood pressure and a systolic blood pressure of the subject are each used as the response variable.

8. A blood pressure measurement method, comprising:
by a computer,
detecting a cuff pressure received by a cuff configured to be wound around an arm of a measurement subject from the arm;
generating a first waveform image that includes a first image of a first pulse waveform acquired from a time-series change in the cuff pressure and indicating a change in pressure brought by pulsation of a blood flow flowing through an artery of the measurement subject, and that indicates the cuff pressure with features of the first image; and
acquiring a blood pressure of the measurement subject from a calculation model by inputting the first waveform image to the calculation model, the calculation model constructed by machine learning in which a second waveform image that includes a second image of a second pulse waveform acquired from a time-series change in the cuff pressure regarding a subject and indicating a change in pressure brought by pulsation of a blood flow flowing through an artery of the subject, and that indicates the cuff pressure with features of the second image is used as an explanatory variable, and a blood pressure of the subject is used as a response variable.

9. A blood pressure measurement program for causing a computer to execute:
detecting a cuff pressure received by a cuff configured to be wound around an arm of a measurement subject from the arm;
generating a first waveform image that includes a first image of a first pulse waveform acquired from a time-series change in the cuff pressure and indicating a change in pressure brought by pulsation of a blood flow flowing through an artery of the measurement subject, and that indicates the cuff pressure with features of the first image; and
acquiring a blood pressure of the measurement subject from a calculation model by inputting the first waveform image to the calculation model, the calculation model constructed by machine learning in which a second waveform image that includes a second image of a second pulse waveform acquired from a time-series change in the cuff pressure regarding a subject and indicating a change in pressure brought by pulsation of a blood flow flowing through an artery of the subject, and that indicates the cuff pressure with features of the second image is used as an explanatory variable, and a blood pressure of the subject is used as a response variable.

10. A learning model construction method, comprising:
by a computer,
detecting a cuff pressure received by a cuff configured to be wound around an arm of a subject from the arm;
generating a waveform image that includes an image of a pulse waveform acquired from a time-series change in the cuff pressure and indicating a change in pressure brought by pulsation of a blood flow flowing through an artery of the subject, and that indicates the cuff pressure with features of the image; and
constructing a learning model by machine learning in which the waveform image is used as an explanatory variable, and a blood pressure of the subject is used as a response variable.

11. A learning model construction program for causing a computer to execute:
detecting a cuff pressure received by a cuff configured to be wound around an arm of a subject from the arm;
generating a waveform image that includes an image of a pulse waveform acquired from a time-series change in the cuff pressure and indicating a change in pressure brought by pulsation of a blood flow flowing through an artery of the subject, and that indicates the cuff pressure with features of the image; and
constructing a learning model by machine learning in which the waveform image is used as an explanatory variable, and a blood pressure of the subject is used as a response variable.
